# EUROPEAN PATENT SPECIFICATION

(11) **EP 0 715 850 B1**
(45) Date of publication and mention of the grant of the patent: **10.11.2004**
(21) Application number: 95308890.3
(22) Date of filing: 07.12.1995
(51) Int. Cl.: A61K 31/401, A61P 1/16

(54) **Use of proline and/or derivatives as an antihepatitis agent**
Verwendung von Prolin und/oder Derivaten als Mittel gegen Hepatitis
Utilisation de la proline et/ou les dérivés comme agent anti-hépatite

(30) Priority: 07.12.1994 JP 30391794
(43) Date of publication of application: 12.06.1996
(73) Proprietor: Ajinomoto Co., Inc., Tokyo 104 (JP); Japan Science and Technology Agency, Kawaguchi-shi, Saitama 332-0012 (JP)
(72) Inventor: Mori, Masato, Yokohama-shi, Kanagawa, 236 (JP); Torii, Kunio, Tokyo, 153 (JP)
(74) Representative: Calamita, Roberto

(56) References cited:
- PHARMACOL BIOCHEM BEHAV, NOV 1995, 52 (3) P509-15, UNITED STATES, HAWKINS RL ET AL 'Proline, ascorbic acid, or thioredoxin affect jaundice and mortality in Long Evans cinnamon rats.'
- FED. PROC., 1984, 43/13 (2815-2820), USA, UITTO J. ET AL 'Pharmacological inhibition of excessive collagen deposition in fibrotic diseases'
- DATABASE WPI Section Ch, Week 9421 Derwent Publications Ltd., London, GB; Class B03, AN 94-173652 & JP-A-06 116 144 ( KIMURA S) , 26 April 1994
- DATABASE WPI Section Ch, Week 7914 Derwent Publications Ltd., London, GB; Class B05, AN 79-26847B & JP-A-54 026 324 ( MORISHITA PHARM KK) , 27 February 1979
- DATABASE WPI Section Ch, Week 8614 Derwent Publications Ltd., London, GB; Class B03, AN 86-091210 & JP-A-61 037 733 ( OTSUKA PHARM KK) , 22 February 1986
- FASEB JOURNAL, vol. 7, 1993 pages 596-591, LEE, J. ET AL 'Proline-mediated enhancement of hepatocyte function in a collagen gel sandwich culture configuration'
- IN VITRO CELLULAR AND DEVELOPMENTAL BIOLOGY, vol. 21, no. 2, February 1985 pages 121-124, HOUCK, K.A. ET AL 'Proline is required for the stimulation of DNA synthesis in hepatocyte cultures by EGF'
- THE BIOCHEMICAL JOURNAL, vol. 273, no. 1, 1991 pages 57-62, BAQUET, A. ET AL 'Comparison of the effects of various amino acids on glycogen synthesis, lipogenesis and ketogenesis in isolated rat hepatocytes'
- BIOCHEMICAL AND BIOPHYSICAL RESEARCH COMMUNICATIONS , vol. 122, no. 3, 1984 pages 884-891, NAKAMURA, T. ET AL 'L-proline is an essential amino acid for hepatocyte growth in culture'

## Description

### FIELD OF THE INVENTION

The present invention relates to an antihepatitis agent. More particularly, the present invention relates to an antihepatitis agent having an excellent effect on improvement of hepatitis including fulminant hepatitis.

### PRIOR ART

Hepatitis is a diffuse inflammatory disease of the liver and is classified into acute hepatitis, fulminant hepatitis, subacute hepatitis, long lasting hepatitis, and chronic hepatitis in terms of seriousness and stadium. In terms of cause, known types of hepatitis include viral hepatitis caused by hepatitis virus, alcoholic hepatitis caused by drinking, drug induced hepatitis caused by a medical drug, heavy metal poisoning hepatitis caused by a heavy metal, and autoimmune hepatitis caused by autoimmunity mechanism. Symptoms include hypertrophy of the liver at a stadium of strong inflammation, oppressive pain and hitting pain of the liver and jaundice. The liver gradually contracts in fulminant hepatitis and subacute hepatitis. According to the disease becomes chronic with liver fibrosis, the liver scleroses, finally resulting in denaturation of cells and necrosis.

At all events, the liver is an organ taking charge of functions indispensable for maintaining homeostasis of a biological body. Any hindrance thereof, however slight, causes many troubles in daily life, and progress of disease has a serious risk on the survival of individuals. Conventionally, however, there is known no clearly effective drug against hepatitis. When symptom is slight, however, improvement of amino acid balance in blood and supply of nutrimenL are attempted via a dietary cure, or depending upon the extent of progress of disease, through administration of a mixed infusion containing amino acid.

Various mixed infusions containing amino acid of various general prescriptions are known, including, for example, an amino acid infusion for cancer patients is disclosed in Japanese Patent Provisional Publication No. S55-35,049. To approximate the plasma amino acid composition of a sound person, this infusion contains all the eight types of essential amino acid. For amino acid infusion for cirrhosis patients, with a view to approximating amino acid in blood in a patient to that of a sound person through supply of nutrient, it is proposed to administer amino acid in short (Japanese Patent Provisional Publication No. S56-27,493), and an infusion which contains much branch chain amino acid such as leucine, isoleucine and valine and has low contents of tyrosine and phenylalanine is proposed (Japanese Patent Provisional Publication No. S55-36,457 and Japanese Patent Provisional Publication No. S54-26,324). For eliminating hindrance in liver substance synthesizing function, there is known an amino acid composition which contains cystine, cysteine or a mixture thereof, alanine, aspartic acid and glycine in respective prescribed qualities (Japanese Patent Publication No. H06-67,831).

Additionally, it is also known that one or two kinds of specific amino acid are effective for prevention or improvement of liver dysfunction. For example, following are disclosed that alanine and/or glutamine are effective for improvement of alcoholic diseases (Japanese Patent Provisional Publication No. S63-54320); alanine and/or glutamine increase hepaLocytes and thereby promote the regeneration of liver (Japanese Patent Provisional Publication No. H5-229940); proline or lysin has an effect for prevention of alcoholic liver disfunction by decreasing an amount of acetaldehyde during alcohol ingestion (Japanese Patent Provisional Publication No. H6-116144); a combined use of alanine and ornithine has an effect for decreasing the dysfunction of liver tissue and has anti-alcoholic liver dysfunction effect (Japanese Patent Provisional Publication No. S61-50917); and at least one of alanine, glutamine and ornithine is effective for treatment of viral hepatitis, drug induced hepatitis and fulminant hepatitis (Japanese Patent Provisional Publication No. H5-221858).

On the other hand, the present inventors administered 15 kinds of aqueous amino acid solution to Long Evans Cinnamon (LEC) rats, which caused spontaneous development of hepatitis, cirrhosis or hepatoma for free selective ingestion. As a result, proline and branching amino acids (valine, leucine, isoleucine) were selectively ingested, and found that development of hepatitis in LEC rats could be inhibited by free ingestion or administration of proline solution.

However, abnormal accumulation of copper in the liver caused by congenital copper metabolism abnormality is considered to be a cause of a pathological change in the liver in LEC rats and is different from the general hepatitis model in many points.

Consequently, the antihepatitis effect of proline could not be proved.

Derwent abstract 86-091210 of JP-A-6137733 (Otsuka Pharm. KK) discloses combinations of EGF and proline for treating hepatitis. However, in view of Nakamura et al 1984 (Biochemical and Biophysical Research Communications vol. 122, nr. 3, p.884-891), the use of proline as a single agent against hepatitis would not be indicated (page 890, paragraph 2, lines 6-13).

### SUMMARY OF THE INVENTION

The present invention has an object to provide a novel antihepatitis agent which permits alleviation of the extent of dysfunction of the liver due to hepatitis, in spite of a simple composition, by proving the antihepatitis effect of proline.

The present invention provides an antihepatitis agent containing proline, a pharmaceutically acceptable salt thereof, or any mixture of two or more thereof as the effective ingredient.

According to the present invention, there is provided an antthepatitis agent which contains proline as the effective ingredient, and is effective for inhibition of progress of symptoms and improvement of patient's condition. More particularly, the antihepatitis agent of this invention is effective for treatment of heavy metal poisoning hepatitis, alcoholic hepatitis and hepatitis followed the fulminent hepatitis. Because the effective ingredient is a single kind of amino acid, it is very easy to prepare, store and prescribe same, without any adverse side effect.

### BRIEF DESCRIPTION OF THE DRAWINGS

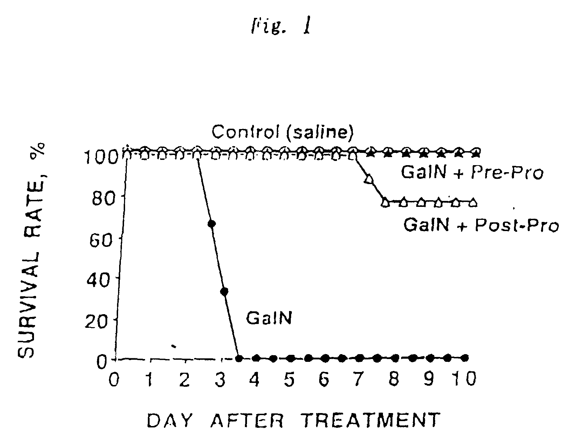

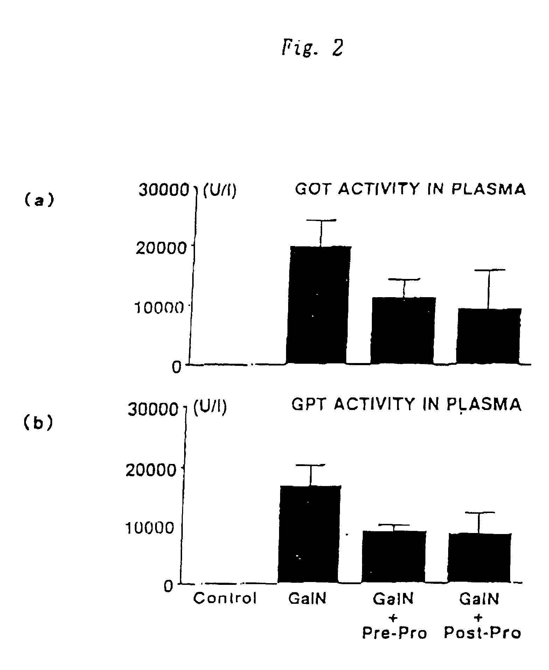

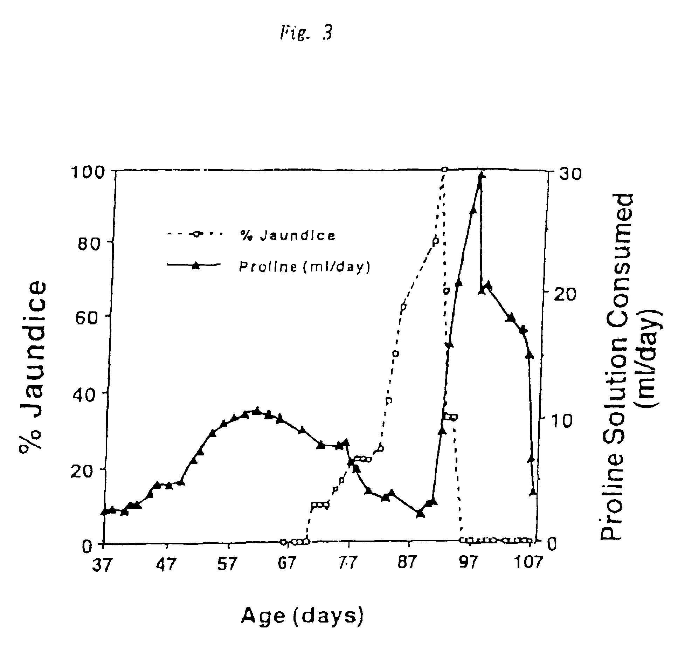

Fig. 1 is a graph illustrating the survival rate of rats suffering galactosamine induced fulminant hepatitis in ten days after administration of galactosamine. Fig. 2 (a) is a graph illustrating GOT activity of GOT in blood of rats suffering galactosamine induced fulminant hepatitis; and (b) is a graph illustrating activity of GPT in blood thereof. Fig. 3 a graph showing a daily change of proline solution consumed by LEC rats and of % jaundice of the rats.

### DETAILED DESCRIPTION OF THE INVENTION

Proline, and pharmaceutically acceptable salts of proline (hereafter these be denoted as "prolines") can be converted into medical drugs in any of such forms as powder, solid and liquid. It is also possible to add, as required, a pharmaceutically acceptable lipid, sugar, vitamins and minerals. These additives may be uniformly mixed with prolines to form a medical drug. When adding trace ingredients such as vitamins, uniform mixing may be achieved by once dissolving prolines in water, adding vitamins to the resultant solution for dissolution, and lyophilizing the mixture. The mixture may be dissolved in aqueous solution of carboxymethylcellulose (CMC).

The amount of administration of the antihepatitis agent of the present invention should be within a range of from 1 to 10 g per adult person, or more preferably, from 1.5 to 4.0 g. This amount may appropriately be adjusted in response to the condition, the status of nutrient, the age and the body weight of the patient.

Now, the antihepatitis agent of the present invention will be described in further detail with reference to the results of experiments carried out on antihepatitis effect or prolines as examples.

### Example 1

Because of the similarity of pathological findings to those of human hepatitis and a large number of patients, experiments were carried out on the effect of administration of proline on the survival rate of rats suffering galactosamine induced fulminant hepatitis commonly used as a hepatitis model.

### (1) Procedure:

Fisher strain rats (male; eight weeks of age; body weight: 160 to 190 g) were divided into four groups each comprising nine rats. Rats of three groups were administered with 30% galactosamine hydrochloride-saline through intraperitoneal administration (1.4 g/kg body weight) to form fulminant hepatitis models (GalN), and only saline was administered to the remaining group to form a control group. Oral administration (2.0 g/kg body weight) of proline (Pro) to the GalN rats (two groups) was accomplished one hour prior to, and after the lapse of 12 and 24 hours from, administration of galactosamine for the pre-administration (Pre-Pro) group, and after the lapse of 6, 12 and 24 hours from administration of galactosamine for the post-administration (Post-Pro) groups.

The survival rate of the individual rats was observed for ten days after administration of galactosamine at intervals of 12 hours. For 12 hours before and after administration of galactosamine, the rats were fasted, and from 12 hours prior to administration of galactosamine through six hours thereafter, the rats were caused to freely ingest 10% glucose aqueous solution.

### (2) Results:

The results of this test are shown in Fig. 1. As is clear from Fig. 1, while, upon the lapse of ten days after administration of galactosamine, the proline non-administration group (GalN) showed a survival rate of 0% (0/9), the post-administration group gave a survival rate of 78% (7/9) and the pre-administration group, 100% (9/9), thus revealing a remarkable improvement of survival rate brought about by administration of proline.

### Example 2

The effect of administration of proline on transamylase activity in blood of rats suffering galactosamine induced fulminant hepatitis was investigated.

### (1) Procedure:

Blood was sampled from each of the four groups of rats (GalN, Control, Pre-Pro, and Post-Pro) of Example 1, upon the lapse of 36 hours after administration of galactosamine, and activity of transamylase in blood (glutamic-oxaloacetic acid transamylase: GOT; glutamic-pyruvic acid transamylase: GPT) was measured in accordance with the conventional method.

### (2) Results:

The results of this test are shown in Fig. 2. As is clear from Fig. 2, the sharp increase in the concentration of GOT and GPT in blood under the effect of development of fulminant hepatitis was largely inhibited by the administration of proline. Increase or decrease in GOT and GPT is commonly utilized as an indicator showing the extent of hindrance of the liver functions, and the remarkable inhibiting effect thereon proved the antihepatitis effect of proline.

### Example 3

An effect of proline was investigated by using LEC rat which spontaneously develops hepatitis, cirrhosis and hepatoma.

### (1) Procedure:

Weanlings of LEC rats (seven weeks of age: food deprived for one week before testing) were divided into 2 groups, and copper deficiency feed and normal feed were presented to the groups, respectively. The rats of 2 groups were further divided into 3 groups: group of free drinking of proline solution (0.2M of proline in distilled water); group of oral administration of proline solution (135-150 mg of proline/day); and group of free drinking of water. Death rate of 6 groups of rats were measured. A relationship between the amount of proline solution consumed by rats and the degree of jaundice of the rats was also measured.

### (2) Results:

The results of death rate are shown in Table 1. An increase of death rate was observed in the normal feed group compared with the copper deficiency feed group. This result indicates that the rats in normal feed group accumulate copper in liver due to congenital copper metabolism abnormality of LEC rats. Moreover, the proline-ingested or -administrated rats significantly decrease death rate compared with water drinking group. Especially, in the group of copper deficiency feed + proline, survival of all rats was observed.

**Table 1**

| Groups | Death rate |
|---|---|
| Copper deficiency feed + Proline (oral administration) | 0 % |
| Copper deficiency feed + Proline (free drinking) | 0 % |
| Copper deficiency feed + Water (free drinking) | 43 % |
| Normal feed + Proline (oral administration) | 57 % |
| Normal feed + Proline (free drinking) | 85 % |
| Normal feed + Water (free drinking) | 100 % |

Next, the relationship between the amount of consumed proline and the degree of jaundice is shown in Fig. 3. Rats ingested more proline in response to increase of jaundice which is typical symptome of hepatitis, and thereby diminished the jaundice.

It is confirmed from the above results that proline is effective for prevention or inhibition of hepatitis due to copper accumulation on liver.

## Claims

1. Use of proline or a pharmaceutically acceptable salt thereof as the single effective ingredient in the manufacture of a medicament for the treatment of hepatitis.

2. Use as claimed in claim 1 wherein the medicament further comprises a pharmaceutically acceptable lipid, sugar, vitamins and minerals.

## Patentansprüche

1. Verwendung von Prolin oder einem pharmazeutisch annehmbaren Salz davon als einziger wirksamer Bestandteil zur Herstellung eines Medikaments zur Behandlung von Hepatitis.

2. Verwendung nach Anspruch 1, wobei das Medikament ferner ein pharmazeutisch annehmbares Lipid, Zucker, Vitamine und Mineralien enthält.

## Revendications

1. Utilisation de proline ou d'un sel acceptable en pharmacie de celle-ci, en tant qu'ingrédient actif unique, dans la fabrication d'un médicament destiné au traitement de l'hépatite.

2. Utilisation selon la revendication 1, dans laquelle le médicament comprend en outre un lipide, un sucre, des vitamines et des minéraux, acceptables en pharmacie.
